(19)

Europäisches
Patentamt
European
Patent Office
Office européen
des brevets

(11) **EP 4 759 355 A1**

(12) **EUROPEAN PATENT APPLICATION**

(43) Date of publication:
**17.06.2026   Bulletin 2026/25**

(21) Application number: **24218730.0**

(22) Date of filing: **10.12.2024**

(51) International Patent Classification (IPC):
***A61N 5/10*** (2006.01)

(52) Cooperative Patent Classification (CPC):
**A61N 5/103**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Siemens Healthineers International AG 6312 Steinhausen (CH)**

(72) Inventors:
- **KUUSELA, Esa**
  **02320 Espoo (FI)**
- **HYVÖNEN, Heini**
  **00560 Helsinki (FI)**

(74) Representative: **HKW Intellectual Property PartG mbB**
**Theresienhöhe 12**
**80339 München (DE)**

(54) **COMPUTER-IMPLEMENTED METHOD, APPARATUS, COMPUTER PROGRAM PRODUCT AND SYSTEM FOR RADIATION TREATMENT PLANNING**

(57)     The present invention relates to a computer-implemented method, a computer-implemented device, a system and a computer program for determining a radiation treatment plan for a patient comprising determining a boundary condition constraining the radiation treatment plan; determining a first expected isodose surface in a body of the patient for a first specified radiation dose level based on the determined boundary condition, the first expected isodose surface comprising a plurality of dose point, defining a volume of interest in the body of the patient, the volume of interest being delimited by plurality of delimitation points, determining a plurality of metric distances between at least a selection of the plurality of dose points and of at least a first selection of the plurality of delimitation points and selecting a metric distance of the plurality of metric distances based on the boundary condition.

FIG 1A

EP 4 759 355 A1

**Description**

The present invention relates to a computer-implemented method, a computer-implemented apparatus, a system and a computer program product for radiation treatment planning.

The increasing number of cancer diagnoses every year increased the demand for modern treatment facilities that are capable of providing an increasing number of patients access to adequate treatment schemes.

A commonly used treatment method for cancer therapy is radiation therapy wherein tumor cells are at least locally irradiated by a suitable radiation beam such as, e.g., X-rays, gamma rays, protons, heavy ions, etc. A critical aspect of radiation therapy is the radiation dose required to effectively harm malign tumor cells while ensuring that the radiation dose deployed in healthy tissue is minimized (e.g., to avoid the risk of causing carcinogenic cell defects as a result of the radiation process) while keeping the overall duration (e.g., the total time required to deliver a pre-defined radiation dose to a malign tissue of a patient) of the radiation process as low as possible.

An actual radiation therapy is often carried out based on a preceding radiation treatment planning during which a dose to be delivered to a patient is determined for a plurality of settings of a radiation apparatus (e.g., various directions under which a radiation beam may impinge onto a patient, an energy of the radiation beam and/or doses for certain locations of the body of the patient which should be irradiated).

A radiation apparatus may comprise a radiation particle source (e.g., for electrons, protons, heavy ions, X-rays or gamma rays, etc.), an acceleration stage and/or may comprise a gantry for directing the accelerated radiation particles onto a patient.

Even though radiation therapy is frequently applied in hospitals and/or dedicated practices, radiation therapy remains a cost-intensive method for cancer therapy. This cost-intensiveness is, e.g., driven by the required medical staff (e.g., for planning the radiation therapy and/or executing the actual radiation therapy), the costs for the required hardware (e.g., a radiation apparatus and potential replacement parts), maintenance costs, energy costs for operating the hardware, etc.

The generation of a radiation treatment plan is often based on an optimizer algorithm which may receive a patient geometry (e.g., based on computed tomography (CT) images) and one or more objectives based on which the optimizer may determine an optimized radiation treatment plan. Said objectives may, e.g., be referred to as boundary conditions which may define that a certain portion of the body of the patient should at least receive a certain radiation dose level. This process is also referred to as inverse-planning rather than expressly specifying how a certain dose should be delivered to a patient. A boundary condition may be referred to herein as any kind of cost function. In such a case, the optimizer algorithm may find an optimized solution that satisfies the defined boundary conditions and that minimizes the cost function, respectively.

The optimizer-based radiation treatment planning approach may be based on defining a certain volume (e.g., a volume of interest) in the body of the patient (e.g., a volume that should be irradiated or a volume which should not be irradiated) followed by a definition of a certain metric related to the dose distribution (e.g., a maximum dose ($D_{max}$), a minimum dose ($D_{min}$), a mean dose ($D_{mean}$), etc. to be achieved at a certain location in the body of the patient).

It may be required during radiation treatment planning to not only focus on the defined volume of interest as such but to also take a surrounding area of the defined volume of interest into account. In this context, it may be seen desirable to evaluate how close a 40 Gy isodose is from a spinal cord of the patient (and to, e.g., push the isodose surface away from the organ). In some cases, it may be seen beneficial to specify that a 50% dose level does not extend unnecessarily far from a planned target volume (PTV). In some cases, some stereotactic radiosurgeries (SRS) and stereotactic body radiotherapies (SBRT) cases may be set boundary conditions which may lead to a "volcano" dose distribution where PTV is hottest (e.g., highest with respect to a radiation dose level) in a narrow region (e.g., outside a clinical target volume (CTV) and/or a gross tumor volume (GTV)). In multi-level targets (e.g., in simultaneous integrated booster (SIB treatments) it may sometimes be required to control a radiation dose distribution just outside a higher-dose target. In an example, in SIB cases, a higher dose target area (e.g., a booster area) may be prescribed to for example 70 Gy. The lower dose area (prescription may, e.g., be 56 Gy) may be larger and may contain OARs. To ensure a control treatment toxicity for malicious tissue, the 70 Gy dose may be required to be limited as well as possible within the higher dose target area. This may imply a decrease of the dose of 70 Gy to 56 Gy in a short distance within the respective target area, e.g., within < 1 cm.

Many radiation treatment planning processes are based on the definition of helper structures (e.g., by a planner or by the optimizer algorithm). However, the definition of helper structures may be seen disadvantageous in many applications. When helper structures (e.g., ring structures) are defined, they are always defined for a certain distance from, e.g., the defined volume of interest and they are solely suitable for evaluating metrics within the specified distance. In an example, it may be seen beneficial to push a 40 Gy isodose surface away from the spine as much as possible. However, the usage of a helper structure may require a definition of a distance from the surface of the organ and may thus require a heuristic knowledge of the distance which counters the idea of finding an optimization radiation treatment plan. If, e.g., $D_{max}$ for a 2 cm margin structure is used to specify this desire, there is no possibility to inform the optimizer what should be done next if the margin structure is reached, that is, it may be impossible to define that it would be desirable to push a 40 Gy dose outside of, e.g., a 2.5 cm margin and thus further away from the volume of interest or that, if it is not possible to push the 40 Gy dose outside of the ring structure, there is no difference whether the 40 Gy isodose is close to the spine in a certain direction or just barely inside the ring.

What is more, evaluating several radiation treatment plan variants with different helper structures (e.g., different ring structures) is difficult as there is often no clear way to compare different dose value histograms (DVH) associated with different boundary conditions.

Thus, there is currently no procedure which allows a radiation treatment planning that is sufficiently advanced to overcome the aforementioned drawbacks.

Therefore, there is a need to improve the currently performed radiation treatment planning procedures.

It is one object to provide means for improving the radiation treatment planning in an efficient manner.

According to a first aspect, a computer-implemented method for determining a radiation treatment plan for a patient is suggested. The computer-implemented method may comprise determining a boundary condition constraining the radiation treatment plan and determining a first expected isodose surface in a body of the patient for a first specified radiation dose level based on the determined boundary condition, the first expected isodose surface comprising a plurality of dose points. Moreover, the computer-implemented method may comprise defining a volume of interest in the body of the patient, the volume of interest being delimited by a plurality of delimitation points and determining a plurality of metric distances between at least a selection of the plurality of dose points and of at least a first selection of the plurality of delimitation points. The computer-implemented method may further comprise selecting a metric distance of the plurality of metric distances based on the boundary condition.

The boundary condition may refer to an objective associated with the radiation treatment plan. In some examples, more than one boundary condition may be determined.

The boundary condition may be determined from a user input. That is, a planner of the radiation treatment planning may provide the boundary condition by means of a user-interface (e.g., a keyboard input, a mouse-based input, a touch-screen-based input, a spoken input and/or any other suitable input).

An isodose surface may be referred to as a geometric surface in a three-dimensional space of the body of the patient along which a constant radiation dose level may be expected. This surface is defined by a plurality of dose points where the same radiation dose level may be achieved. The determination of an isodose surface may be carried out by calculating and visualizing the distribution of the radiation dose level within the body of the patient, based on pre-defined radiation parameters and considering boundary conditions. These boundary conditions can, for example, include that the isodose surface is required to maintain or maximize a certain distance from a defined volume (e.g., an organ-at-risk or a clinical target volume) to achieve optimal therapeutic effects while minimizing undesirable side effects.

The plurality of delimitation points may define a shell encompassing the volume of interest. In some examples, the volume of interest may define a volume in which respectively encompassed tissue of the body of the patient should not receive a radiation treatment. In some examples, the volume of interest may define a volume in which respectively encompassed tissue of the body of the patient should receive a radiation treatment.

The metric distance may be referred to as an indicator (e.g., in mm, cm, m, etc.) that quantifies a spatial separation of, e.g., the first expected isodose surface and the volume of interest. The metric distance may be measured between a respective dose point of the isodose surface and a respective point (e.g., a delimitation point) of the volume of interest.

Based thereon, a more versatile radiation treatment planning procedure may be facilitated, e.g., in the sense of a distance-to-dose metric for inverse planning of a radiation treatment plan. That is, a boundary condition defined for the radiation treatment planning is not necessarily bound to a certain distance as no helper structure needs to be defined. Instead, it may be facilitated that a planner may define a desired radiation dose level distribution outside the volume of interest without any need for specifying respective distances (e.g., hard-coded distances). This may allow the optimizer to seek solutions wherein an optimality of the radiation treatment plan depends on a distance of a certain dose level from a defined volume of interest.

More specifically, the computer-implemented method proposed herein may outperform helper structure-based (such as, e.g., ring structures) radiation treatment planning procedures. This may, e.g., apply in conditions of failure when a boundary condition was not met (e.g., if a hot region may be pushed as far away as possible from the defined volume of interest even if it has not been pushed away from the volume of interest as far as originally desired). Moreover, classical helper structures may also be outperformed in situations in which a boundary condition was met as the improvements in radiation treatment planning may, e.g., allow to push a hot region even further away from a desired region as it would be possible based on a helper structure. The latter applies as the definition of a helper structure may only allow the definition that a certain isodose surface should at least be located 2 cm away from the volume of interest. If this boundary condition is met, no further optimization may apply. However, the optimization of finding a certain location of an isodose surface may not be limited to a certain pre-defined distance but may be optimized in a more global and generic manner. Therefore, an improved radiation treatment planning procedure may be provided.

In an embodiment, the computer-implemented method may further comprise providing a radiation apparatus configuration for controlling a radiation apparatus adapted to provide a radiation process to the patient in accordance with the selected metric distance.

The radiation apparatus configuration may comprise all data that is required to set the radiation apparatus into a state in which it may provide the radiation process in accordance with the metric distance and/or the determined boundary condition.

In some examples, the computer-implemented method may further comprise executing the radiation process based on the radiation apparatus configuration.

In a further embodiment, the boundary condition may define a maximum distance of the first expected isodose surface (for a certain radiation dose level) from the volume of interest or that the first expected isodose surface (for a certain radiation dose level) should be as far apart from the volume of interest as possible or that the first expected isodose surface (for a certain radiation dose level) should be as close to the volume of interest as possible.

In some examples, the maximum distance or the minimum distance may refer to a distance of 1 cm to 10 cm, 2 cm to 9 cm, 3 cm to 8 cm, 4 cm to 7 cm, 5 cm to 6 cm or any other suitable distance. In some examples, the maximum distance or the minimum distance may refer to a distance of less than 1 cm. In some examples, the maximum distance or the minimum distance may refer to a distance of more than 10 cm.

This may provide a more generic and versatile radiation treatment planning procedure and may allow to tailor the radiation treatment planning more closely to the actual needs of a patient.

In a further embodiment, the determining of the plurality of metric distances may further comprise determining a plurality of metric distances between a plurality of dose points that is associated with a radiation dose level that is higher than the radiation dose level associated with the first specified radiation dose level of the first expected isodose surface and of at least a second selection of the plurality of delimitation points.

The plurality of dose points that is associated with the radiation dose level that is higher than the radiation dose level associated with the specified radiation dose level of the first expected isodose surface may be located further apart from the volume of interest as compared to the first expected isodose surface. In some examples, the plurality of dose points that is associated with the radiation dose level that is higher than the radiation dose level associated with the specified radiation dose level of the first expected isodose surface may be located closer to the volume of interest as compared to the first expected isodose surface.

In some examples, the determining of the plurality of metric distances may further comprise determining a plurality of metric distances between a plurality of dose points that are associated with a radiation dose level that is less than the radiation dose level associated with the specified radiation dose level of the first expected isodose surface and of at least a second selection of the plurality of delimitation points.

This may efficiently support the determination of at least a second isodose surface which may match the determined boundary condition more closely.

In a further embodiment, the computer-implemented method may further comprise determining a maximum metric distance of the plurality of metric distances based on the determined boundary condition or may further comprise determining a minimum metric distance of the plurality of metric distances based on the determined boundary condition.

The determining of a maximum metric distance may comprise a systematic comparison of each of the plurality of metric distances with any other one of the plurality of metric distances and an evaluation which of the compared metric distances is larger. Based thereon, the computer-implemented method may comprise determining a list of metric distances in ascending order (e.g., with respect to an ascending metric distance).

This may support the determination of those points of the plurality of dose points and the plurality of delimitation points that lie closest to each other or that lie furthest apart from each other. As these points may define the points that may be seen most critical for an evaluation whether a certain isodose surface fulfills the determined boundary condition, a clearer assessment may be made whether the first expected isodose surface fulfills the determined boundary condition.

In a further embodiment, the computer-implemented method may further comprise iteratively repeating the steps of determining the first expected isodose surface based on the determined maximum or minimum, determining the plurality of metric distances and selecting the metric distance to obtain an expected isodose surface that is in accordance with the determined boundary condition.

In some examples, the computer-implemented method may be executed at least N times, with $N \in \mathbb{N}$ .

By iteratively repeating the steps of determining the first expected isodose surface, determining the plurality of metric distances and selecting the metric distance, an iterative and thus improved determination of the first expected isodose surface may be supported.

In a further embodiment, the computer-implemented method may further comprise deriving a gradient from the determined maximum metric distance or the determined minimum metric distance, the gradient being indicative of a change of the radiation dose level per metric deviation from the first expected isodose surface.

The gradient may indicate by how much a (expected) radiation dose level may change along a certain spatial direction if a certain location in the body of the patient is considered that may be displaced from the first expected isodose surface.

In some examples, the gradient may be defined as a two-dimensional vector (referenced to a Cartesian coordinate system) that may indicate by how much the radiation dose level may change if a location in the body of the patient is considered that is, e.g., displaced by a certain metric distance along an x-direction and/or a y-direction of the Cartesian coordinate system. In some examples, the gradient may be defined as a three-dimensional vector (referenced to a respective Cartesian coordinate system).

Based on the deriving of the gradient, an iterative execution of the computer-implemented method to obtain an optimized first expected isodose surface may efficiently be supported as the gradient may support a heuristic decision how a spatial shift of the initially defined isodose surface along a certain direction may alter the expected radiation dose. Therefore, a number of required iterations of the execution of the computer-implemented method to arrive at a first expected isodose surface that may closely meet the desired determined boundary condition may thus efficiently be supported. Therefore, a more efficient radiation treatment planning procedure may be provided.

In a further embodiment, the computer-implemented method may further comprise determining a second expected isodose surface in the body of the patient for a second specified radiation dose level that is higher than the first specified radiation dose level, the second expected isodose surface defining a plurality of intermediate dose points that are arranged in between the first expected isodose surface and the second expected isodose surface and determining the plurality of metric distances between the plurality of dose points and the plurality of individual delimitation points.

The second expected isodose surface may be associated with a location that lies further away from the volume of interest as compared to the first expected isodose surface. Alternatively, the second expected isodose surface may be associated with a location that lies closer from the volume of interest as compared to the first expected isodose surface.

In some examples, the computer-implemented method may further comprise determining the plurality of metric distances between at least a selection of the intermediate dose points and the volume of interest.

This may efficiently provide a more versatile radiation treatment planning procedure as the boundary condition may not be limited to a single first expected isodose surface only (which may be associated with a respective single (first) radiation dose level) but may be extended to a range of radiation dose levels wherein the range of radiation dose levels may be defined by the radiation dose level associated with the first expected isodose surface and the radiation dose level associated with the second isodose surface. As a result, the optimization of the computer-implemented method may seek for an isodose surface that meets the determined boundary condition and which isodose surface is associated with a radiation dose level within the aforementioned range.

In a further embodiment, a radiation dose level may increase with a pre-defined proportionality from the first specified radiation dose level associated with the first expected isodose surface to the second specified radiation dose level associated with the second expected isodose surface.

In some examples, the pre-defined proportionality may be a linear proportionality.

Alternatively, the radiation dose level may increase in quadrature or cubically from the specified radiation dose level associated with the first expected isodose surface to the specified radiation dose level associated with the second expected isodose surface. Alternatively, the radiation dose level may increase based on another proportionality between the first expected isodose surface and the second expected isodose surface.

By providing the first expected isodose surface and the second expected isodose surface such that a radiation dose level defined therebetween may increase with a pre-defined proportionality, a more efficient determination of a radiation treatment plan may be supported. More specifically, the pre-defined proportionality may support a tailored subsequent iteration step for determining a subsequent first expected isodose surface. Consequently, a more efficient and more targeting determination of a radiation treatment plan may be facilitated.

In a further embodiment, the determining of the plurality of metric distances may further comprise setting a metric distance of the plurality of metric distances to a default value if a respective determined metric distance exceeds a pre-defined threshold.

In some examples, the default value may be 0. In some examples, the default value may be 1. In some examples, the default value may be -1. Alternatively, it may also be possible that the default acquires any other suitable default value.

In some examples, the default value may also be applicable if the respective determined metric distance undercuts a pre-defined threshold.

Setting a specific determined metric to a certain default value may avoid that certain dose points that may be considered as outliers may falsify the determination of the respective metric distance. What is more, setting respective metric distances that exceed or undercut a pre-defined threshold to a default value may effectively allow a weighting of those dose points that may, e.g., be not of relevance (e.g., as they exceed or undercut the respective pre-defined threshold) for the calculation of a metric distance.

In a further embodiment, the determining of the plurality of metric distances may further comprise setting a metric distance of the plurality of metric distances to a value less than zero if a dose point, used to determine the respective metric, lies within the volume of interest.

In some examples, it may also be possible to set a metric distance of the plurality of metric distances to a value larger than zero if a dose point, used to determine the respective metric, lies within the volume of interest.

In some examples, the metric distance may be set to a value of -1 if a dose point used to determine the respective metric lies within the volume of interest.

By setting a metric which includes a dose point located in the volume of interested to a value of less than zero, its contribution to the calculation may be weighted. This may allow to, e.g., exclude the calculation of such a metric from, e.g., the determination of a maximum metric distance or a minimum metric distance.

In a further embodiment, the volume of interest may be an organ-at-risk, a clinical-target-volume, an organ-at-risk-volume and/or a planned target volume.

An "*organ-at-risk*" (OAR) may be referred to as a critical organ or tissue within a patient's body that is sensitive to radiation and thus must be carefully considered during the planning and delivery of radiation therapy. The goal may be to minimize the radiation dose to these structures to prevent any undue damage or significant side effects while effectively treating the target area. This may involve a precise calculation and mapping to ensure that the radiation dose remains within safe limits for these vulnerable organs, while still delivering the necessary therapeutic dose to the target area.

A "*clinical target volume*" (CTV) may be referred to as a volume that encompasses a gross tumor volume (GTV) along with any potential microscopic malignant disease. This volume may be identified based on clinical judgment and imaging studies, ensuring that the entirety of the area at risk, including subclinical disease, is adequately treated. The CTV may be a critical component in radiation therapy, as it may account for the spatial uncertainty in the precise location of microscopic disease and may aim to ensure comprehensive coverage and effective dose delivery to target malignant cells while minimizing exposure to surrounding healthy tissues.

An "*organ-at-risk volume*" may be referred to as a specific volume within the body of the patient that may contain critical organs or tissues which are sensitive to radiation. These organs-at-risk (OAR) shall be carefully considered during the planning and delivery of radiation therapy. A goal may be seen to minimize radiation exposure to these structures to prevent undue damage or significant side effects while ensuring effective treatment of the target area.

A "*planned target volume*" (PTV) may be referred to as a specified volume within the patient's body that encompasses the clinical target volume (CTV) and an additional margin. This margin may account for potential variations and uncertainties in patient positioning, organ motion, and other factors that might influence the precise delivery of the radiation dose. The PTV may ensure that the prescribed therapeutic dose uniformly covers the entire CTV, including any microscopic malignant disease, despite these uncertainties.

This may support that the determined radiation treatment plan may more closely be tailored to the needs of the patient.

Any embodiment of the first aspect may be combined with any embodiment of the first aspect to obtain another embodiment of the first aspect.

According to a second aspect, a computer program product is suggested. The computer program product may comprise instructions which, when the program is executed by a computer, cause the computer to carry out the computer-implemented method as described herein.

A computer program product, such as a computer program means, may for example be provided or delivered as a storage medium, such as a memory card, USB stick, CD-ROM, DVD, or in the form of a downloadable file from a server on a network. This can be done, for example, in a wireless communication network by transferring a corresponding file with the computer program product or the computer program means.

According to a third aspect, a computer-implemented apparatus for determining a radiation treatment plan for a patient is suggested which may comprise a first determining unit for determining a boundary condition constraining the radiation treatment plan and a second determining unit for determining a first expected isodose surface in a body of the patient for a first specified radiation dose level based on the determined boundary condition, the first expected isodose surface comprising a plurality of dose points. The computer-implemented apparatus may further comprise a defining unit for defining a volume of interest in the body of the patient, the volume of interest being delimited by plurality of delimitation points and a third determining unit for determining a plurality of metric distances between at least a selection of the plurality of dose points and of at least a first selection of the plurality of delimitation points. Moreover, the computer-implemented apparatus may comprise a selecting unit for selecting a metric distance of the plurality of metric distances based on the boundary condition.

In some examples, the determining unit may comprise a communication interface (e.g., Bluetooth, Wireless Fidelity (WiFi), Universal Serial Bus (USB), etc.) by means of which the respective boundary condition may be retrieved from a remote entity such as, e.g., a remote server (e.g., comprising a database).

In some examples, the selecting unit may comprise a user interface (e.g., a (touch-sensitive) screen which may be configured to display the selected distance, a loudspeaker (by means of which the selected distance may be output as a spoken sequence or any other suitable acoustic means), a printer (which may be configured to print the selected distance on a piece of a paper), etc.).

The respective entities, e.g. the first determining unit, the second determining unit, the defining unit, the third determining unit and/or the selecting unit, may be implemented in hardware and/or in software. If said entity is implemented in hardware, it may be embodied as a device, e.g. as a computer or as a processor or as a part of a system, e.g. a computer system. If said entity is implemented in software it may be embodied as a computer program product, as a function, as a routine, as a program code or as an executable object.

In some examples, the boundary condition may also refer to the determining of a weighted sum of individual boundary conditions (e.g., taking into account how certain metrics deviate from goal values).

According to an embodiment, the computer-implemented apparatus may further comprise an execution unit for executing the computer-implemented method as described herein. Moreover, the computer-implemented apparatus may comprise a further execution unit for executing the computer program product as described herein.

The execution unit for executing the computer-implemented method or the further execution unit for executing the computer program may, e.g., comprise a processor (e.g., a CPU) and/or a graphics processing unit (GPA) and/or a tensor processing unit (TPU) and/or a field programmable gate array (FPGA).

In some examples, the computer-implemented apparatus may comprise a providing unit for providing a radiation apparatus configuration for controlling a radiation apparatus adapted to provide a radiation process to the patient in accordance with the selected metric distance.

In some examples, the computer-implemented apparatus may further comprise a radiation process executing unit for executing the radiation process in accordance with the radiation apparatus configuration.

This may support efficient execution of the steps of the method as described herein.

According to a fourth aspect, a system for radiation treatment planning is suggested. The system may comprise the computer-implemented apparatus as described herein. Moreover, the system may comprise the computer program product as described herein.

In some examples, the computer-implemented apparatus and the computer program product may be provided as a single device. Alternatively, the computer-implemented apparatus and the computer program product may be arranged as two separate devices. In the latter case the computer program product may, e.g., be arranged at a remote entity such as, e.g., a remote server, a remote database, etc.

In one or more examples, the functions described may generally be implemented in hardware, software, firmware, or any combination thereof. If implemented in software, the functions may be stored on or encoded as one or more instructions or code on a computer- readable medium. Computer-readable media includes computer storage media. Storage media may be any available media that can be accessed by a computer. By way of example, and not limitation, such computer-readable media can comprise random-access memory (RAM), read-only memory (ROM), electronically erasable programmable ROM (EEPROM), compact disk (CD) ROM (CD-ROM), or other optical disk storage, magnetic disk storage or other magnetic storage devices, or any other medium that can be used to carry or store desired program code in the form of instructions or data structures and that can be accessed by a computer. Disk and disc, as used herein, includes CD, laser disc, optical disc, digital versatile disc (DVD), and floppy disk where disks usually reproduce data magnetically, while discs reproduce data optically with lasers. Combinations of the above should also be included within the scope of computer readable media.

The embodiments and features described with reference to the apparatus of the present invention apply *mutatis mutandis* to the method of the present invention and vice versa.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages will become apparent from the subsequent description and dependent claims, taken in conjunction with the accompanying drawings, in which:

Fig. 1 shows exemplary illustrations of metric distances;

Fig. 2 shows examples of a determination of a metric distance for a range of radiation dose levels;

Fig. 3 depicts an exemplary flow diagram of a computer-implemented method for determining a radiation treatment plan for a patient;

Fig. 4 depicts an exemplary flow diagram of a computer-implemented method for determining a radiation treatment plan for a patient according to another embodiment;

Fig. 5 depicts an exemplary computer-implemented apparatus for determining a radiation treatment plan for a patient; and

Fig. 6 depicts an exemplary system for determining a radiation treatment plan.

In the Figures, reference numerals designate like or functionally equivalent elements, unless otherwise indicated.

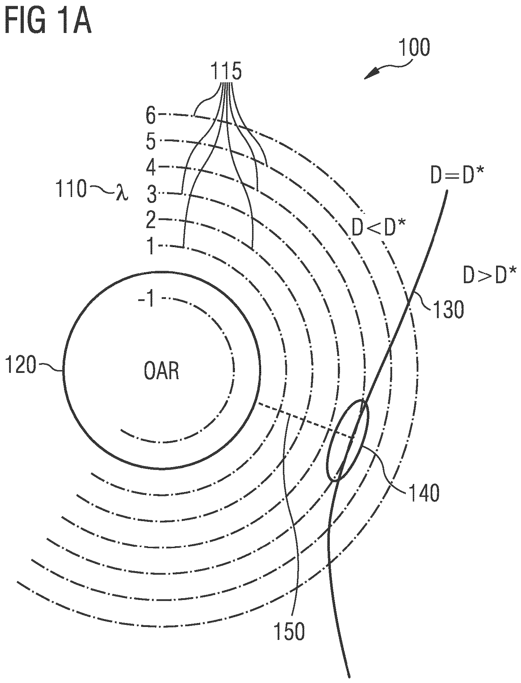

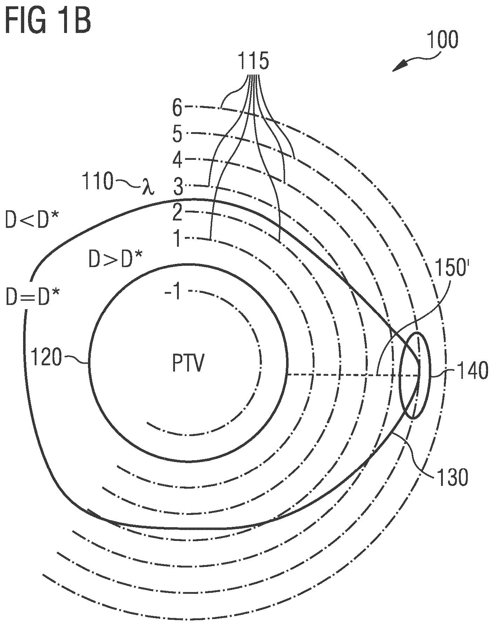

Figs. 1A and 1B show exemplary illustrations 100 of metric distances between a volume of interest and a first expected isodose surface.

More specifically, Fig. 1A depicts an exemplary illustration 100 of a determination of a metric distance 110 between a volume of interest 120 and a first expected isodose surface 130.

The metric distance 110 (expressed by $\lambda(x)$) may be associated with a plurality of concentric circles 115 about the volume of interest 120 wherein a larger radius of one of the concentric circles 115 is associated with a larger distance from the volume of interest 120.

The volume of interest 120 may be encompassed by a plurality of delimitation points.

In the example depicted in Fig. 1A, the volume of interest 120 may be provided as an organ-at-risk (OAR).

The isodose surface 130 may be associated with a certain (defined) radiation dose level D*. The isodose surface 130 may comprise a plurality of dose points that are all associated with a same radiation dose level. The isodose surface 130 may be provided as a first expected isodose surface 130.

Relative to the radiation dose level D*, a radiation dose level D may be defined that is smaller than the radiation dose level D* such that D < D* applies. In the example depicted in Fig. 1A, said radiation dose level D may be associated with respective dose points which lie closer to the volume of interest 120 (and its plurality of delimitation points respectively) than the respective dose points of the first exemplary isodose surface 130.

Relative to the radiation dose level D*, a radiation dose level D may be defined that is larger than the radiation dose level D* such that D > D* applies. In the example depicted in Fig. 1A, said radiation dose level D may be associated with respective dose points which lie further apart from the volume of interest 120 (and its plurality of delimitation points respectively) than the respective dose points of the first exemplary isodose surface 130.

Region 140 depicts an exemplary region of the first exemplary isodose surface 130 where a decrease in dose level may improve the metric distance between the volume of interest 120 and the first exemplary isodose surface 130. That is due to the aspect that an optimization algorithm may be operated most efficiently in a region in which a small change in the dose distribution alters the metric distance such that the optimizer can effectively focus to this region to improve the metric distance. Since a minimum metric distance (expressed by reference numeral 150) is the closest distance between OAR and the isodose surface, the minimum metric distance where a small change in the dose distribution may alter the metric distance (whereas any small change in the isodose surface far away from this point may not change the metric distance), may be located in that region. It may generally be a single point along the isodose surface 130 where an infinitesimal change in the dose distribution may change the metric distance. For numerical reasons, it may be seen beneficial to not only consider a particular single point on isodose surface 130 but a small finite region (e.g., region 140) surrounding the isodose surface 130 wherein a small change of the dose distribution in the region 140 may later the metric distance as described above.

In the example depicted in Fig. 1A, the region 140 may be associated with a minimum metric distance 150 between the volume of interest 120 and the first exemplary isodose surface 130. The minimum metric distance $\overline{\lambda}$ (expressed by the reference numeral 150) of a plurality of determined metric distances $\lambda(x)$ may be expressed as:

$$\overline{\lambda} = \min_{x} \lambda(x), \ when \ D(x) \geq D^{*}.$$

<div align="right">equation 1</div>

In some examples, the metric distance $\lambda(x)$ may be provided as a signed distance (i.e., $\lambda(x)$ may be positive or may be negative). Equation 1 may be used to describe the calculation of the metric distance wherein region 140 is effectively provided as a single point.

Equation 1 may be rephrased to allow for an efficient numerical implementation. Such a numerical implementation may be written as:

$$\overline{\lambda} = \frac{\int dx \, \lambda(x) \, e^{-\lambda(x)\beta} \, \overline{\theta}(D(x)-D^{*})}{\int dx \, e^{-\lambda(x)\beta} \, \overline{\theta}(D(x)-D^{*})}.$$

<div align="right">equation 2</div>

Equation 2 differs from equation 1 in that that the min-operator of equation 1 has been replaced by a softmin approach in which every metric distance 110 (that is $\lambda(x)$) has been taken into a sum (expressed by the integral) with a weight that is proportional to $e^{-\lambda\beta}$, wherein $\beta$ is a factor which indicates how much larger metric distances should be taken into account.

The nominator of equation 2 fulfills the task of providing a normalization of the metric $\bar{\lambda}$ such that an undesired increase of the metric $\bar{\lambda}$ towards infinity is avoided. Equation 2 effectively describes the calculation of a metric distance where the region 140 is provided as a spatially extended region (e.g., as shown in Figs. 1A and 1B).

Moreover, the condition that $D(x) \geq D^*$ is replaced by a smooth version of the step function $\bar{\theta}(x)$, wherein $\bar{\theta}(x)$ may be defined as $\bar{\theta}(x) = 1 - \mathrm{erf}\left(\frac{x}{\gamma}\right)$. Here, $\gamma$ denotes a smoothing factor indicating how an extent by how much other dose radiation dose levels as compared to a specified radiation dose level (e.g., as defined by a boundary condition) should be taken into account.

Based on equation 2, a gradient may be derived around a certain dose point at location x:

$$\nabla_{D(x)}\bar{\lambda} = \frac{e^{-\lambda(x)\beta}\overline{\delta}(D(x)-D^*)}{\int dx' e^{-\lambda(x')\beta}\overline{\theta}(D(x')-D^*)}\left(\lambda(x) - \bar{\lambda}\right).$$

equation 3

Here, x and x' indicate two arbitrary locations within a dose matrix. It may be assumed that a gradient of the dose at location x may be zero if x does not belong to region 140, i.e., if the location association with x lies outside of the region 140. The gradient may be calculated relative to the dose at location x such that it may be representative for the change rate of the metric value if the dose at that location x is changed.

Moreover, $\overline{\delta}$ denotes a smooth version of the delta function which may be obtained from deriving the smoothed step-function $\bar{\theta}(x)$. In case $\bar{\theta}(x) = 1 - \mathrm{erf}\left(\frac{x}{\gamma}\right)$, $\bar{\delta}(x) = \exp\left(-\frac{x^2}{\gamma^2}\right)$ applies.

Fig. 1B depicts an exemplary illustration 100 of a determination of a metric distance 110 between a volume of interest 120 and a first exemplary isodose surface 130.

The metric distance 110 (expressed by $\lambda(x)$) may be associated with a pluraltiy of concentric circles 115 about the volume of interest 120 wherein a larger radius of one of the concentric circles 115 is associated with a larger distance from the volume of interest 120.

In the example depicted in Fig. 1B, the volume of interest 120 may be provided as a planned target volume (PTV).

The isodose surface 130 may be associated with a certain radiation dose level D*. The isodose surface 130 may comprise a plurality of dose points that are all associated with a same radiation dose level.

Relative to the radiation dose level D*, a radiation dose level D may be defined that is smaller than the radiation dose level D* such that D < D* applies. In the example depicted in Fig. 1B, said radiation dose level D may be associated with respective dose points which lie further apart from the volume of interest 120 (and its plurality of delimitation points respectively) than the respective dose points of the first exemplary isodose surface 130.

Relative to the radiation dose level D*, a radiation dose level D may be defined that is larger than the radiation dose level D* such that D > D* applies. In the example depicted in Fig. 1B, said radiation dose level D may be associated with respective dose points which lie closer to the volume of interest 120 (and its plurality of delimitation points respectively) than the respective dose points of the first exemplary isodose surface 130.

Region 140 depicts an exemplary region of the first exemplary isodose surface 130 where a decrease in dose level may improve the metric distance between the volume of interest 120 and the first exemplary isodose surface 130.

In the example depicted in Fig. 1B, the region 140 may be associated with a maximum metric distance 150' between the volume of interest 120 and the first exemplary isodose surface 130. In analogy to equation 1, the maximum metric distance $\bar{\lambda}$ of a pluraltiy of determined metric distances $\lambda(x)$ may be expressed as:

$$\bar{\lambda} = \max_x \lambda(x), \ when \ D(x) \geq D^*.$$

equation 4

As outlined with a view to equation 1, above, also the metric distance $\lambda(x)$ of equation 4 may be provied as a signed distance. In analogy to equation 2, also equation 4 may be rephrased such that it may allow an efficient numerical implementation:

$$\bar{\lambda} = \frac{\int dx\, \lambda(x)\, e^{\lambda(x)\beta}\overline{\theta}(D(x)-D^*)}{\int dx\, e^{\lambda(x)\beta}\overline{\theta}(D(x)-D^*)}.$$

equation 5

For practical reasons, it may be possible to combine the calculation of the metric distance with the definition of a pre-defined value of the metric distance in case a determined distance metric exceeds a pre-defined threshold value. In case the determined metric distance exceeds a certain threshold value, the value of respective metric distance may be set to a same cost value. This may effectively allow to restrict the calculation of the metric distance (e.g., according to one of the equations 2 or 5) to a vicinity of the volume of interest 120 only.

It is further emphasized that in some cases the calculation of the metric distance may also be extended to a calculation of the metric distance for dose points that may lie within the volume of interest 120 (e.g., if the isodose surface 130 at least partially lies within the volume of interest 120). In such cases, a negative value may be assigned to the metric distance.

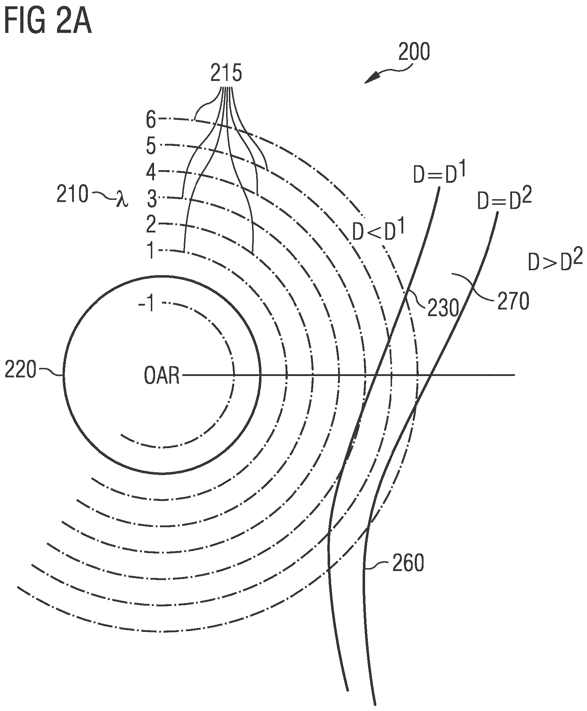

Figs. 2A - 2C depict an example of a determination of a metric distance for a range of radiation dose levels.

More specifically, Fig. 2A depicts an exemplary illustration 200 of a determination of a metric distance 210 between a volume of interest 220 and a first expected isodose surface 230 as well as a second expected isodose surface 260.

A metric distance 210 (expressed by $\lambda(x)$) may be associated with a pluraltiy of concentric circles 215 about the volume of interest 220 wherein a larger radius of one of the concentric circles 215 is associated with a larger distance from the volume of interest 220.

The volume of interest 220 may be encompassed by a plurality of delimitation points.

In the example depicted in Fig. 2A, the volume of interest 220 may be provided as an organ-at-risk (OAR).

In the example depicted in Fig. 2A, the second expected isodose surface 260 is defined in addition to the first expected isodose surface 230. As the first expected isodose surface 230 may be associated with a plurality of dose points (and thus a respective first radiation dose level $D^1$) and as the second expected isodose surface 260 may be associated with a plurality of dose points (and thus a respective second radiation dose level $D^2$), a plurality of intermediate radiation dose points 270 may be defined which may be associated with an intermediate radiation dose level that lies in between the first radiation dose level $D^1$ and the second radiation dose level $D^2$.

In some examples, a radiation dose level associated with a respective radiation dose point may be increased based on a pre-defined proportionality from the first radiation dose level $D^1$ to the second radiation dose level $D^2$.

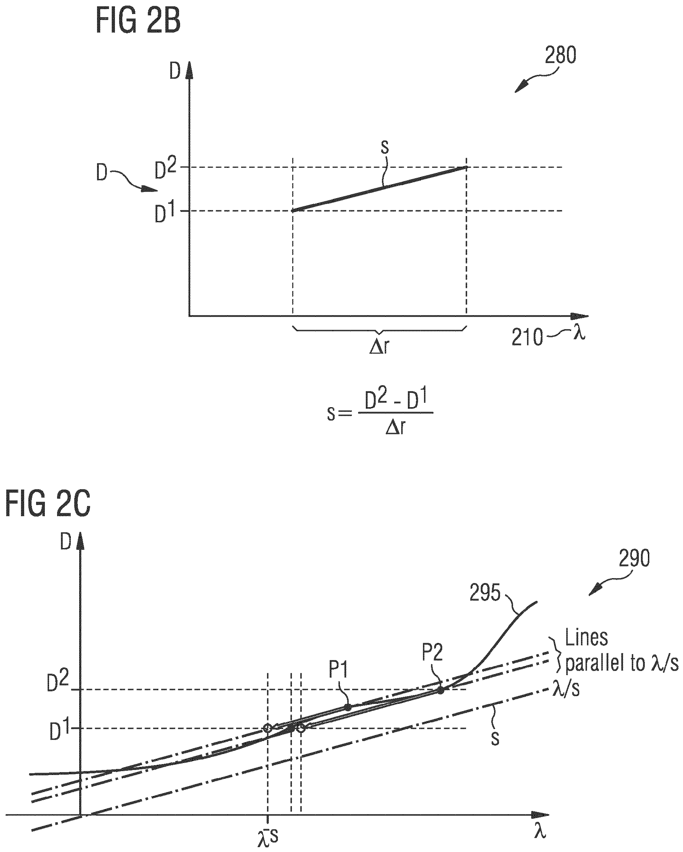

Fig. 2B shows a diagram 280 which depicts an exemplary relationship between a radiation dose level D and a metric distance $\lambda$ (e.g., the metric distance 150). In the depicted example, a linear proportionality between the radiation dose level D and the metric distance 110 is assumed. That is, an increase of the metric distance 110 by a fixed value may be accompanied by a respectively fixed increase of the radiation dose level D. The relationship between the increase of the radiation dose level D and the metric distance 110 may be expressed as a respective slope s of the exemplarily depicted relationship wherein the slope s may be given by:

$$S = \frac{D^2 - D^1}{\Delta r}.$$

equation 6

Here, $\Delta r$ may denote the metric distance difference between a metric distance of respective dose points that are associated with the second expected isodose surface 260 and with the first expected isodose surface 230.

The definition of the first expected isodose surface 230 and of the second expected isodose surface 260 may in particular be seen as beneficial if a planner intends to not solely focus on a single dose level but intends to consider at least a range of doses defined by the interval $[D^1; D^2]$. In such a case, a respective radiation dose level may change as a function of the metric distance within the defined interval.

In some cases, an expected radiation dose level $\hat{D}(\lambda')$ may be obtained, wherein $\lambda'$ denotes an increase of the metric distance 110 compared to the dose point at which the radiation dose level $D^1$ is obtained. Based thereon the metric $\hat{\lambda}(x)$ may be defined

$$\hat{\lambda}(x) = \begin{cases} \lambda(x) - \hat{D}^{-1}(D(x)), & when\ D^1 \leq D(x) \leq D^2 \\ 0 & otherwise \end{cases},$$

equation 7

wherein $\hat{D}(\lambda')$ is invertible. Subsequent to the determination of $\hat{\lambda}(x)$ for each dose point x, a determination of a maximum or minimum distance may be executed, e.g., based on one of the equations 1 or 4.

To support an efficient numerical determination of the metric distance $\hat{\lambda}(x)$, equation 7 may be rephrased as

$$\bar{\lambda} = \frac{\int dx \; \hat{\lambda}(x) \; e^{-\hat{\lambda}(x)\beta}(\theta(D(x)-D^1)\theta(D^2-D(x)))}{\int dx \; e^{-\hat{\lambda}(x)\beta}(\theta(D(x)-D^1)\theta(D^2-D(x)))}.$$

equation 8

In the given context, there is no need to replace the step function $\theta(x)$ with a smoothed approximation, since $\hat{\lambda}(x)$ is a function of D(x) itself.

Fig. 2C shows an exemplary diagram 290 which depicts an exemplary relationship between a radiation dose level D and a metric distance $\lambda$.

In some examples, there may be a pre-defined proportionality between the radiation dose level D and a respective metric distance $\lambda$ which may be expressed by the slope s.

Fig. 2C depicts an exemplary procedure to evaluate different radiation dose levels at different metric distances. This may be executed for each dose point, where $D^1 \leq D(x) \leq D^2$, by shifting each dose point to the radiation dose level $D^1$ associated with the first expected isodose surface 130 by shifting the respective dose point along the pre-defined slope s until the radiation dose level $D^1$ is reached.

More specifically, exemplary radiation dose line 295 may comprise a plurality of radiation dose points (e.g., dose points P1 and P2) which are associated with different radiation dose levels and which are associated with respective different metric distances.

To evaluate at which metric distances dose point P1 may be associated with which radiation dose level, dose point P1 may be shifted parallel to the slope s, e.g., up to the radiation dose level D1 which may be associated with a lower boundary of the defined dose interval $[D^1; D^2]$. A similar procedure may be applicable for dose point P2.

The entities described assigned with reference numerals 210, 215, 220, 230 of Fig. 2 may be equal to the entities assigned with reference numerals 110, 115, 120, 130 and with reference to Fig. 1.

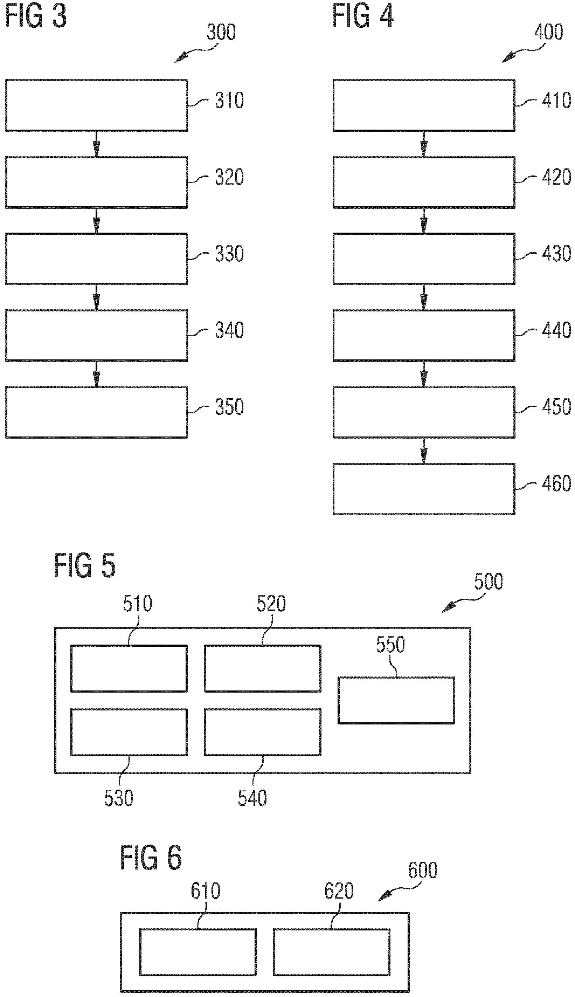

Fig. 3 shows an exemplary computer-implemented method 300 for determining a radiation treatment plan for a patient.

In step 310, a determining a boundary condition constraining the radiation treatment plan may be executed.

In step 320, a determining of a first expected isodose surface in a body of the patient for a first specified radiation dose level based on the determined boundary condition, the first expected isodose surface comprising a plurality of dose points, may be executed. An example for a first expected isodose surface (e.g., the first expected isodose surface 130) is depicted in Figs. 1A, 1B and 2B.

In step 330, a defining of a volume of interest in the body of the patient, the volume of interest being delimited by a plurality of delimitation points, is executed. An example for a volume of interest is, e.g., depicted in Figs. 1A ("OAR", 120), 1B ("PTV", 120) and 2A ("OAR", 220).

In step 340, a determining of a plurality of metric distances between at least a selection of the plurality of dose points and of at least a first selection of the plurality of delimitation points may be executed.

In step 350, a selecting of a metric distance of the plurality of metric distances based on the boundary condition may be executed. The selected metric distance may, e.g., be the minimum metric distance 150 (Fig. 1A) or the maximum metric distance 150' (Fig. 1B).

Fig. 4 shows an exemplary computer-implemented method 400 for determining a radiation treatment plan for a patient according to another embodiment.

The steps 410 - 450 may be identical to steps 310 - 350 as described with reference to Fig. 3, above.

In addition, the computer-implemented method 400 may comprise step 460.

In step 460, a providing of a radiation apparatus configuration for controlling a radiation apparatus adapted to provide a radiation process to the patient in accordance with the selected metric distance may be executed. In some examples, the radiation process may be executed (by the radiation apparatus) according to the radiation apparatus configuration.

Fig. 5 shows an exemplary computer-implemented apparatus 500 for determining a radiation treatment plan for a patient. The apparatus 500 may include a first determining unit 510, a second determining unit 520, a defining unit 530, a third determining unit 540 and a selecting unit 550.

The first determining unit 510 may be configured for determining a boundary condition constraining the radiation treatment plan.

The second determining unit 520 may be configured for determining a first expected isodose surface in a body of the patient for a first specified radiation dose level based on the determined boundary condition, the first expected isodose surface comprising a plurality of dose points.

The defining unit 530 may be configured for defining a volume of interest in the body of the patient, the volume of interest being delimited by plurality of delimitation points.

The third determining unit 540 may be configured for determining a plurality of metric distances between at least a selection of the plurality of dose points and of at least a first selection of the plurality of delimitation points.

The selecting unit 550 may be configured for selecting a metric distance of the plurality of metric distances based on the boundary condition.

Fig. 5 shows an exemplary system 600 for determining a radiation treatment plan for a patient. The system 600 may include a computer-implemented apparatus 610 and a computer program product 620.

Computer-implemented apparatus 610 may be configured as the computer-implemented apparatus 400 as described with reference to Figs. 4 and 5 above.

Computer program product 620 may be configured as described elsewhere herein.

Although the present invention has been described in accordance with preferred embodiments, it is obvious for the person skilled in the art that modifications are possible in all embodiments.

Independent of the grammatical term usage, individuals with male, female or other gender identifies are included with the term.

List of Reference

| | |
|---|---|
| 100 | illustration |
| 110 | metric distance |
| 115 | concentric circles |
| 120 | volume of interest |
| 130 | first expected isodose surface |
| 140 | region |
| 150 | minimum metric distance |
| 150' | maximum metric distance |
| 200 | illustration |
| 210 | metric distance |
| 215 | concentric circles |
| 220 | volume of interest |
| 230 | first expected isodose surface |
| 260 | second expected isodose surface |
| 270 | intermediate radiation dose points |
| 280 | diagram |
| 290 | diagram |
| 295 | radiation dose line |
| 300 | computer-implemented method |
| 310 | step |
| 320 | step |
| 330 | step |
| 340 | step |
| 350 | step |
| 400 | computer-implemented method |
| 410 | step |
| 420 | step |
| 430 | step |
| 440 | step |
| 450 | step |
| 460 | step |
| 500 | computer-implemented apparatus |
| 510 | first determining unit |
| 520 | second determining unit |
| 530 | defining unit |
| 540 | third determining unit |
| 550 | selecting unit |
| 600 | system |
| 610 | computer-implemented apparatus |
| 620 | computer program product |
| D | radiation dose level |
| D1 | radiation dose level associated with first expected isodose surface |

D2    radiation dose level associated with second expected isodose surface
s     slope
P1    dose point
P2    dose point

**Claims**

1.  A computer-implemented method (300; 400) for determining a radiation treatment plan for a patient, comprising:

    determining (310; 410) a boundary condition constraining the radiation treatment plan;
    determining (320; 420) a first expected isodose surface (130; 230) in a body of the patient for a first specified radiation dose level ($D^1$) based on the determined boundary condition, the first expected isodose surface (130; 230) comprising a plurality of dose points;
    defining (330; 430) a volume of interest (120) in the body of the patient, the volume of interest (120) being delimited by a plurality of delimitation points;
    determining (340; 440) a plurality of metric distances (110; 210) between at least a selection of the plurality of dose points and of at least a first selection of the plurality of delimitation points; and
    selecting (350; 450) a metric distance of the plurality of metric distances (110; 210) based on the boundary condition.

2.  The computer-implemented method (400) of claim 1, further comprising:
    providing (460) a radiation apparatus configuration for controlling a radiation apparatus adapted to provide a radiation process to the patient in accordance with the selected metric distance.

3.  The computer-implemented method (300; 400) of one of the preceding claims,
    wherein the boundary condition defines a maximum distance (150') of the first expected isodose surface (130; 230) from the volume of interest (120; 220) or that the first expected isodose surface (130; 230) needs to be as far apart from the volume of interest as possible or that the first expected isodose surface (130; 230) needs to be as close to the volume of interest (120; 220) as possible.

4.  The computer-implemented method (300; 400) of one of the preceding claims, the determining of the plurality of metric distances (110; 210) further comprising:
    determining a plurality of metric distances (110; 210) between a plurality of dose points that are associated with a radiation dose level that is higher than the radiation dose level associated with the first specified radiation dose level ($D^1$) of the first expected isodose surface (130; 230) and of at least a second selection of the plurality of delimitation points.

5.  The computer-implemented method (300; 400) of one of the preceding claims, further comprising:

    determining a maximum metric distance (150') of the plurality of metric distances based on the determined boundary condition; or
    determining a minimum metric distance (150) of the plurality of metric distances based on the determined boundary condition.

6.  The computer-implemented method (300; 400) of claim 5, further comprising:
    iteratively repeating the steps of determining the first expected isodose surface (130; 230) based on the determined maximum (150') or minimum (150), determining the plurality of metric distances (110; 210) and selecting the metric distance to obtain an expected isodose surface that is in accordance with the determined boundary condition.

7.  The computer-implemented method (300; 400) of one of the claims 5 or 6, further comprising:
    deriving a gradient from the determined maximum metric distance (150') or the determined minimum metric distance (150), the gradient being indicative of a change of the radiation dose level per metric deviation from the first expected isodose surface (130; 230).

8.  The computer-implemented method (300; 400) of one of the preceding claims, further comprising:

    determining a second expected isodose surface (260) in the body of the patient for a second specified radiation dose level ($D^2$) that is higher than the first specified radiation dose level ($D^1$), the second expected isodose surface (260) defining a plurality of intermediate dose points that are arranged in between the first expected isodose surface (130; 230) and the second expected isodose surface (260); and

determining the plurality of metric distances (110; 210) between the plurality of dose points and the plurality of individual delimitation points.

9. The computer-implemented method (300; 400) of claim 8,

wherein a radiation dose level increases with a pre-defined proportionality from the first specified radiation dose level ($D^1$) associated with the first expected isodose surface (130; 230) to the second specified radiation dose level ($D^2$) associated with the second expected isodose surface (260).

10. The computer-implemented method (300; 400) of one of the preceding claims, the determining of the plurality of metric distances (110; 210) further comprising:

setting a metric distance of the plurality of metric distances (110; 210) to a default value if a respective determined metric distance exceeds a pre-defined threshold.

11. The computer-implemented method (300; 400) of one of the preceding claims, the determining of the plurality of metric distances (110; 210) further comprising:

setting a metric of the plurality of metric distances (110; 210) to a value less than zero if a dose point, used to determine the respective metric, lies within the volume of interest (120; 220).

12. The computer-implemented method (300; 400) of one of the preceding claims,

wherein the volume of interest (120; 220) is an organ-at-risk, a clinical-target-volume, an organ-at-risk-volume and/or a planned target volume.

13. A computer program product comprising instructions which, when the program is executed by a computer, cause the computer to carry out the computer-implemented method according to one of the claims 1-12.

14. A computer-implemented apparatus (500) for determining a radiation treatment plan for a patient, comprising:

a first determining unit (510) for determining a boundary condition constraining the radiation treatment plan;
a second determining unit (520) for determining a first expected isodose surface (130; 230) in a body of the patient for a first specified radiation dose level ($D^1$) based on the determined boundary condition, the first expected isodose surface (130; 230) comprising a plurality of dose points;
a defining unit (530) for defining a volume of interest (120; 220) in the body of the patient, the volume of interest being delimited by plurality of delimitation points;
a third determining unit (440) for determining a plurality of metric distances (110; 210) between at least a selection of the plurality of dose points and of at least a first selection of the plurality of delimitation points; and
a selecting unit (550) for selecting a metric distance of the plurality of metric distances (110; 210) based on the boundary condition.

15. The computer-implemented apparatus (400) of claim 14, further comprising:

an execution unit for executing the computer-implemented method according to one of the claims 1-12 and/or a further execution unit for executing the computer program product according to claim 13.

## FIG 1A

FIG 1B

FIG 2A

# FIG 2B

280

$$s = \frac{D^2 - D^1}{\Delta r}$$

# FIG 2C

290

## FIG 3

300

310
320
330
340
350

## FIG 4

400

410
420
430
440
450
460

## FIG 5

500

510  520  550  530  540

## FIG 6

600

610  620

Europäisches
Patentamt

European
Patent Office

Office européen
des brevets

## EUROPEAN SEARCH REPORT

**Application Number**

EP 24 21 8730

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/099151 A1 (SULLIVAN ALAN [US] ET AL) 12 April 2018 (2018-04-12) * paragraphs [0060] - [0090], [0108] - [0119] * * figures 2-7 * | 1-15 | INV. A61N5/10 |
| X | US 2016/303398 A1 (ERIKSSON KJELL [SE]) 20 October 2016 (2016-10-20) * paragraphs [0040] - [0043], [0048] * * figure 3 * | 1-3,5, 12-15 | |
| A | US 2020/306559 A1 (KUUSELA ESA [FI] ET AL) 1 October 2020 (2020-10-01) * paragraphs [0007], [0042] - [0072] * * figures 7,8 * | 1-15 | |

**TECHNICAL FIELDS SEARCHED (IPC)**

A61N

The present search report has been drawn up for all claims

| Place of search | Date of completion of the search | Examiner |
|---|---|---|
| Munich | 30 April 2025 | Kretschmann, Jana |

EPO FORM 1503 03.82 (P4C01)

## ANNEX TO THE EUROPEAN SEARCH REPORT
## ON EUROPEAN PATENT APPLICATION NO.

EP 24 21 8730

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2025

| Patent document cited in search report | Publication date | Patent family member(s) | | Publication date |
|---|---|---|---|---|
| US 2018099151 A1 | 12-04-2018 | TW | 201818996 A | 01-06-2018 |
| | | US | 2018099151 A1 | 12-04-2018 |
| | | WO | 2018070109 A1 | 19-04-2018 |
| US 2016303398 A1 | 20-10-2016 | CN | 106029170 A | 12-10-2016 |
| | | EP | 2983781 A1 | 17-02-2016 |
| | | JP | 6483704 B2 | 13-03-2019 |
| | | JP | 2016540598 A | 28-12-2016 |
| | | US | 2016303398 A1 | 20-10-2016 |
| | | WO | 2015090459 A1 | 25-06-2015 |
| US 2020306559 A1 | 01-10-2020 | CN | 113646038 A | 12-11-2021 |
| | | EP | 3946584 A1 | 09-02-2022 |
| | | US | 2020306559 A1 | 01-10-2020 |
| | | WO | 2020200849 A1 | 08-10-2020 |

EPO FORM P0459

For more details about this annex : see Official Journal of the European Patent Office, No. 12/82